(19) Europäisches Patentamt
European Patent Office
Office européen des brevets

(11) Publication number: 0 435 221 A2

(12) # EUROPEAN PATENT APPLICATION

(21) Application number: 90125273.4

(22) Date of filing: 21.12.90

(51) Int. Cl.5: C07D 303/32, C12P 17/02, A61K 31/335, //(C12P17/02, C12R1:465)

(30) Priority: 27.12.89 JP 336256/89

(43) Date of publication of application: 03.07.91 Bulletin 91/27

(84) Designated Contracting States: AT BE CH DE DK ES FR GB GR IT LI LU NL SE

(71) Applicant: HOECHST JAPAN LIMITED
10-16, 8-chome, Akasaka, Minato-ku
Tokyo(JP)

(72) Inventor: Senda, Shunji
7-22 Minamidai 3-chome
Kawagoe-shi, Saitama-ken(JP)
Inventor: Seto, Haruo
Ueno-machi 100
Hachiohji-shi, Tokyo(JP)

(74) Representative: Becker, Heinrich Karl Engelbert, Dr. et al
HOECHST AKTIENGESELLSCHAFT Central Patent Department P.O. Box 80 03 20
W-6230 Frankfurt am Main 80(DE)

(54) **New benzanthracene compound and a process for preparing the same.**

(57) A benzanthracene compound of the formula:

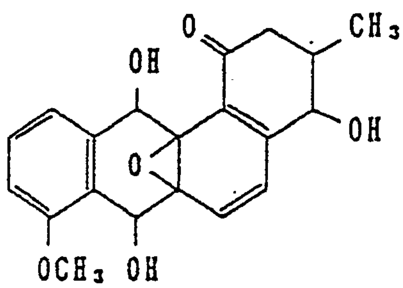

...... 1

a process for preparing the same and its use in a pharmaceutical preparation.

EP 0 435 221 A2

# NEW BENZANTHRACENE COMPOUND AND A PROCESS FOR PREPARING THE SAME

[Field of the Invention]

The present invention relates to new benzanthracene compound useful as an anti-tumor antibiotic, and to a process for preparing the same.

[Background of the Invention]

Prior Art

There are several benzanthracene antibiotics which have hitherto been known, for example, X-14881 D [J. of Antibiot. 35:1627(1982) 38:1270(1985)] of the following structural formula, SF 2315 B.[J. of Antibiot. 41:835, 843(1988)], and the like.

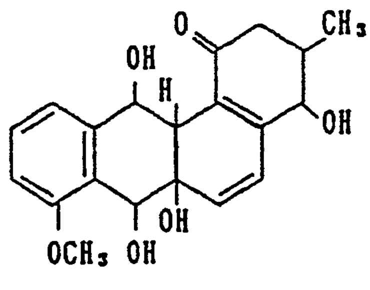

Purpose of the Invention

The object of the invention is to provide a new benzanthracene compound other than the above mentioned antibiotics, also useful as an antitumor agent, and to provide a process for preparing the same.

[Summary of the Invention]

The inventors have now surprisingly found that specific strain belonging to the genus streptomyces produces a product which inhibits the proliferation of mouse leukemia L-1210 cells. The inventors have succeeded in isolating the active component of said product for further study, and identified it as a new anthracene compound having the following structural formula 1:

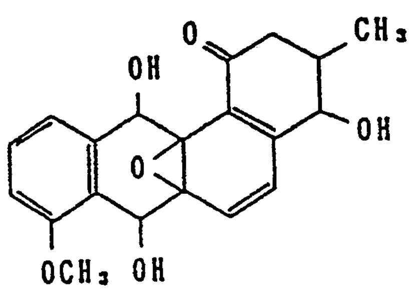

...... 1

and designated as Compound 683.

Figure 1 shows IR(KBr) spectrum of Compound 683.

Figure 2 shows $^1$H-NMR(in DMSO-$d_6$) spectrum of Compound 683.

Figure 3 shows $^{13}$C-NMR(in DMSO-$d_6$) spectrum of Compound 683.

The physicochemical properties of Compound 683 of the present invention are as follows:

(1) Color and shape: white powder;

(2) Elementary analysis: C, 67.04%; H, 5.71%; N, 0%;

(3) Molecular weight: 356;

(4) Molecular formula: $C_{20}H_{20}O_6$;
(5) Melting poing: 204-206° C(decomposition);
(6) Specific rotation: +170° (c=0.023, methanol);
(7) Uv spectrum λmax in methanol: 282nm(ε 7,430), 295nm(shoulder, ε 6,500);
(8) IR spectrum: shown in Fig. 1;
(9) $^1$H-NMR spectum: shown in Fig. 2;
(10) $^{13}$C-NMR spectrum: shown in Fig. 3.

The NMR specta of Compound 683 are similar to those of the above described X-14881 D, suggesting that Compound 683 has the same skeleton as X-14881 D. However, $^1$H-NMR data of X-14881 D shows the presence of peaks at 2.47ppm (12a-H) and 4.10ppm (6a-OH), while Compound 683 does not. Moreover, a peak at 48.2ppm(C-12a) in $^{13}$C-NMR data of X-14881 D is not found in Compound 683, while a unique peak at 67.6ppm is found in Compound 683. From these data, Compound 683 was presumed to be the epoxy derivative of X-14881 D.

The entire structure of Compound 683 has been investigated based on the several types of NMR analyses and found to have the above depicted formula 1.

The present inventon further relates to a process for preparing Compound 683; which comprises culturing Compound 683-producing microorganism belonging to the genus streptomyces, and recovering Compound 683 from the culture. Any strain may be used as Compound 683-producing microorganism according to the present process so far as it is capable of producing sufficient amount of Compound 683 to recover from the culture.

One example of microorganisms capable of producing new antibiotic Compound 683 of the present invention is the Y-83,30683 strain belonging to the genus streptomyces. The microbiological properties of this strain are as follows:

(1) Morphological properties:

The substrate mycelium grows extensively.

The aerial mycelium grows well on oatmeal-agar, glycerol-asparagin-agar, and the like, and the sporulation is abundant. The spore chains of the aerial mycelium are substantially linear.

(2) Growth on various media:

The growth of the Y-83,30683 strain on various types of media are shown in the following Table.

| Medium | Growth | Aerial Mycelium | Soluble Pigment |
|---|---|---|---|
| Yeast-Malts-Agar (ISP No.2) | Normal | Normal | ++ |
| Oatmeal-Agar (ISP No.3) | Good | Abundant | + |
| Starch-Mineral Salt -Agar (ISP No.4) | Good | Abundant | ± |
| Glycerol-Asparagin -Agar (ISP No.5) | Good | Abundant | ++ |
| Peptone-Yeast-Iron -Agar (ISP No.6) | Poor | Poor | + |
| Tyrosine-Agar (ISP No.7) | Normal | Normal | ++ |

(3) Physiological properties:
① Growth temperature:

The Y-83,30683 strain grows at a temperature ranging from 20° C to 40° C on starch-mineral salt-agar medium, and grows well at 27° C to 37° C. It does not grow at 15° C or 50° C.

② Gelatine liquefaction: positive
③ Hydrolysis of starch: positive
④ Reduction of a nitrate: negative
⑤ Peptonization of skimmed milk: positive Coagulation of skimmed milk: negative
⑥ Formation of melanin-like pigment: positive (4) Utilizability of carbon source( on Pridham and Gottlieb's Basal Medium):

Utilizable carbon sources include D-glucose, L-arabinose, glycerol, L-Rhamnose, D-xylose, D-mannitol, Raffinose, D-fructose.

Sucrose and i-inositol are not utilizable.

The above mentioned Y-83,30683 strain was deposited with the Fermentation Research Institute of Agency of Industrial Science and Technology and assigned accession number Bikoken-kinki No.11146-(FERM P-11146).

The Y-83,30683 strain and their mutants (spontaneous mutants, and artificially generated mutants produced by exposure to UV light, X-ray, chemical substances such as nitrosoguanidine, and the like), as well as any other strains belonging to the genus streptomyces and capable of producing Compound 683 of the present invention may be used in the present invention.

In the present process, any of the Compound 683-producing microorganisms may be cultured on a conventional medium containing nutrients which said microorganisms can utilize. Carbon sources which may be mentioned include glucose, starch syrup, glycerol, dextrin, sucrose, starch, molasses, animal and vegetable oils, etc. Suitable nitrogen sources are meat extracts, peptone, yeast extract, ammonium salts, nitrate salts, urea, and the like. In addition, mineral salts capable of producing ions such as sodium, potassium, calcium, magnesium, iron, manganese, cobalt, copper, chloride, phosphate, sulfate, or other ions may also be added, if necessary.

The present process may be conducted under conventional aerobic conditions. For example, reciprocating or rotary shaking culture, aerating spinner culture, solid culture, and the like may be effected. If prevention of foaming is required during culture, anti-foaming agents such as silicone oil, surface active

agents, etc., may be added usually at a concentration of 0.1 to 0.01 % by weight. The incubation is conducted usually at 20-37° C, preferably at 26-30° C. The maximal accumulation of Compound 683 of the present invention in the culture may be attained usually after 2 to 10 days of culture in cases of shaking culture and aerating spinner culture, depending on the type of the medium and the temperature employed.

Compound 683 may be isolated and purified from the culture and mycelium thus obtained, by conventional methods such as extraction with a solvent, column chromatography, recrystallization, etc.

Compound 683 of the present invention, as well as the structurally related X-14881 D, was tested for inhibitory effect on the proliferation of mouse leukemia L-1210 cells(1 x $10^6$ cells/ml). The 50% inhibitory concentration values thereof are as follows.

| | $IC_{50}$ (μg/ml) |
|---|---|
| Compound 683 | 3 |
| X-14881 D | > 100 |

From the above data, it is evident that Compound 683 possesses excellent anti-tumor activity. The compound of the present invention may be used as an anti-tumor agent, alone or in association with a pharmaceutically acceptable carrier, or in a complex having an antibody, a polymer, etc., bound thereto.

[Example]

The invention is further illustrated, but in no way limited, by the following Example.

The compositions of the seed medium and the production medium used are shown in Tables 1 and 2, respectively. The pH of the individual media was adjusted to 7.3 before sterilization.

Table 1

Composition of the Seed Medium

| | |
|---|---|
| Soy flour | 1 % |
| Glucose | 2 % |
| Yeast extract | 0.5 % |
| Sodium chloride | 0.5 % |
| Calcium carbonate | 0.2 % |
| $KH_2PO_4$ | 0.05 % |
| $MgSO_4 \cdot 7H_2O$ | 0.025 % |
| $FeSO_4 \cdot 7H_2O$ | 0.002 % |
| $ZnSO_4 \cdot 7H_2O$ | 0.001 % |
| $MnSO_4 \cdot H_2O$ | 0.0005 % |
| $CuSO_4 \cdot 5H_2O$ | 0.0002 % |
| $CoCl_2 \cdot 6H_2O$ | 0.0001 % |

Table 2

Composition of the Production Medium

| | |
|---|---|
| Glycerol | 1.5 % |
| Glucose | 1 % |
| Trypton | 0.5 % |
| Sodium chloride | 0.5 % |
| Calcium carbonate | 0.2 % |
| $KH_2PO_4$ | 0.05 % |
| $MgSO_4 \cdot 7H_2O$ | 0.025 % |
| $FeSO_4 \cdot 7H_2O$ | 0.002 % |
| $ZnSO_4 \cdot 7H_2O$ | 0.001 % |

| | |
|---|---|
| $MnSO_4 \cdot H_2O$ | 0.0005 % |
| $CuSO_4 \cdot 5H_2O$ | 0.0002 % |
| $CoCl_2 \cdot 6H_2O$ | 0.0001 % |
| $(NH_4)_2MoO_4 \cdot 4H_2O$ | 0.00001 % |

500ml Erlenmeyer flask containing 100ml of the above mentioned seed medium was sterilized for 20 minutes at 121° C, inoculated with 1-2 platinum loops of a slant culture of Y-83,30683 strain and cultured for 4 days at 27° C with shaking, to give a seed culture. 200ml of the seed culture was used to inoculate 7 l jar fermenter containing 4 l of the production medium which had previously been sterilized at 121° C for 20 minutes, and culturing was conducted at 200 rpm and at an aeration rate of 5 l/min at a temperature of 27° C, with occasional addition of an anti-foaming agent Desmophen [Hoechst, A. G., West Germany, Type 3600], if necessary. After 5 days of culturing, the culture thus obtained was centrifuged to separate mycelia from the culture broth.

The mycelia thus collected were extracted with acetone-methanol 1:1 mixture by soaking therein for 24 hours and then filtering. The extract thus obtained was concentrated in vacuo, and the pH of the residue was adjusted to 6.5 and extracted with ethyl acetate. The ethyl acetate extract was concentrated in vacuo and the resultant residue was left to stand at room temperature for 24 hours to give a precipitate. The precipitate was collected by filtration, dissolved in dimethyl sulfoxide, diluted with methanol-water 1:1 mixture, and subjected to preparative high performance liquid chromatography on Unisil Q C18 column [Gasukuro Kogyo Ltd., Tokyo, Japan, 22.2 x 300 mm, particle size 5 μm]. Fractions containing the active component were combined and concentrated in vacuo to dryness to give approximately 80 mg of purified Compound 683.

The strain Y-83,30683 has been redeposited according to the Budapest Treaty under Accession Number FERM BP-3139 at the Fermentation Research Institute (FRI), 1-3, Higashi 1-chome, Tsukuba-shi, Ibaraki-ken 305, Japan.

## Claims

1. A benzanthracene compound of the formula:

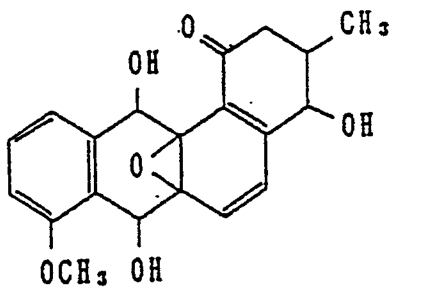
...... 1

2. A process for preparing a benzanthracene compound, which comprises culturing a microorganism belonging to the genus streptomyces and capable of producing a benzanthracene compound of the formula:

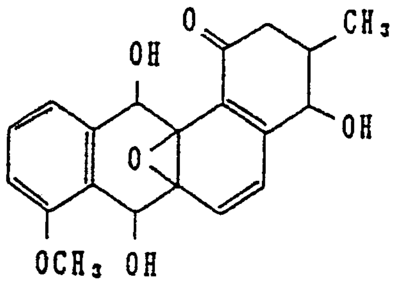

...... 1

and recovering said compound from the culture.

3. Use of the compound of claim 1 in a pharmaceutical preparation.

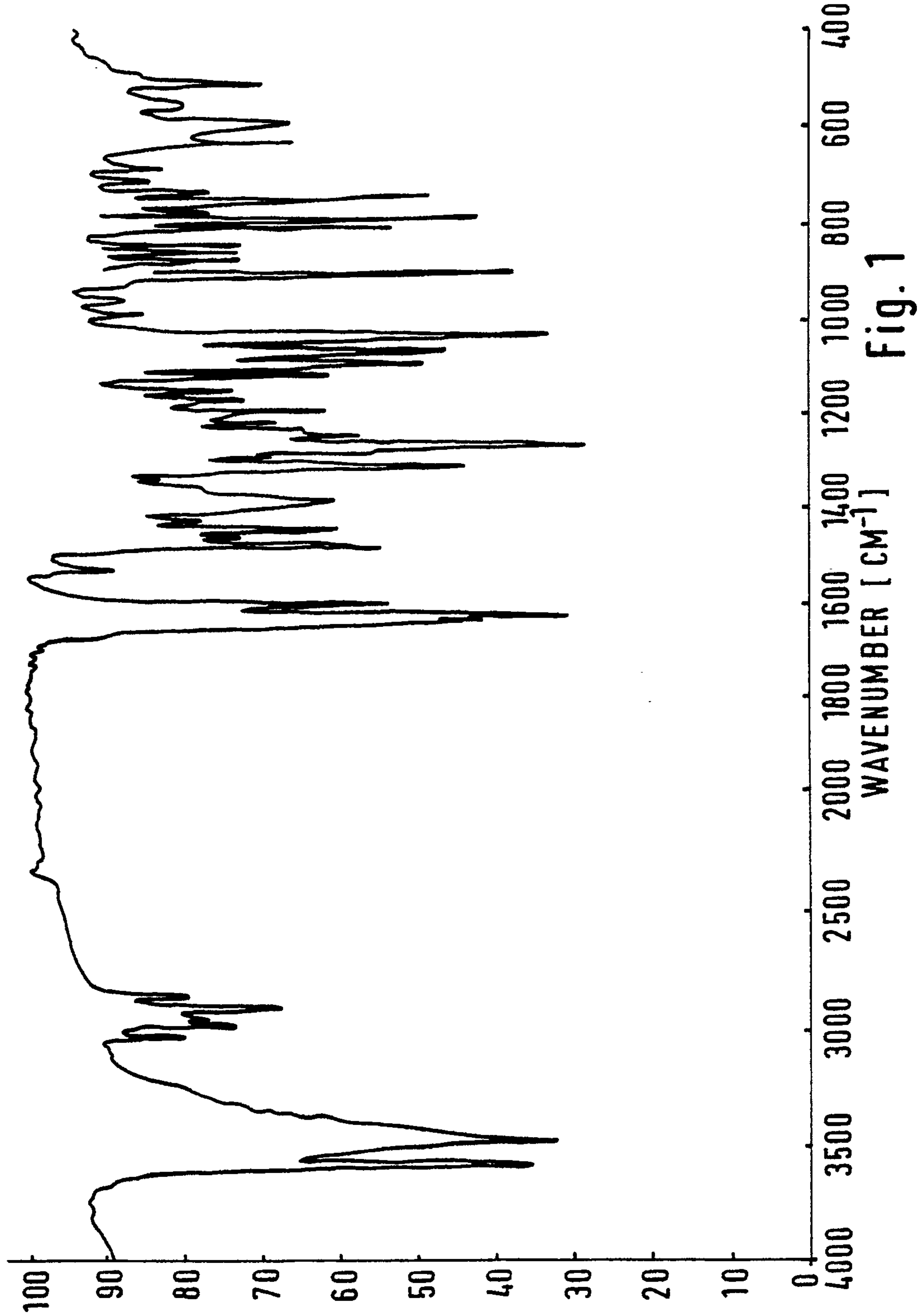
100
90
80
70
60
50
40
30
20
10
0
4000
3500
3000
2500
2000
1800
1600
1400
1200
1000
800
600
400
WAVENUMBER [CM-1]

Fig. 1

Fig. 2

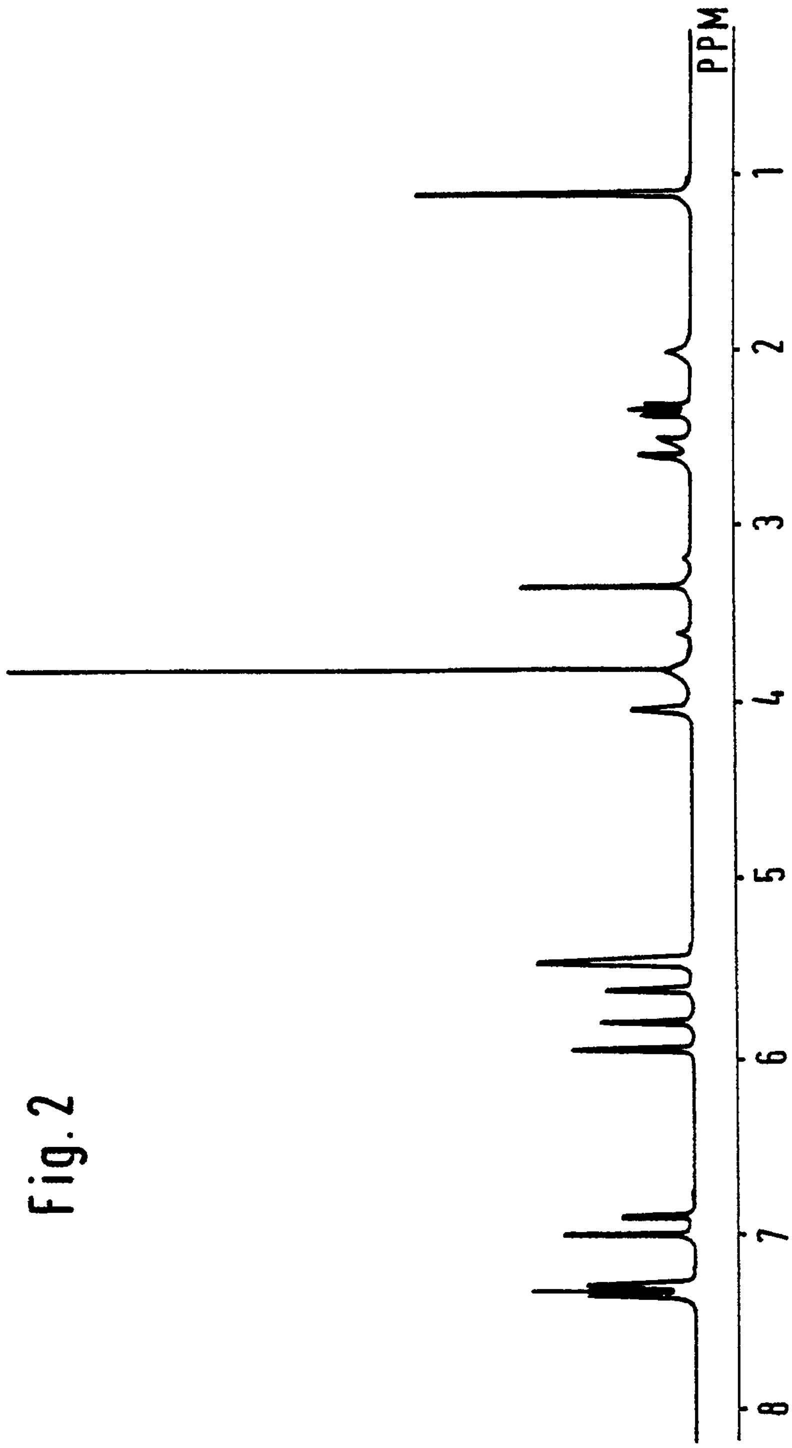

Fig. 3

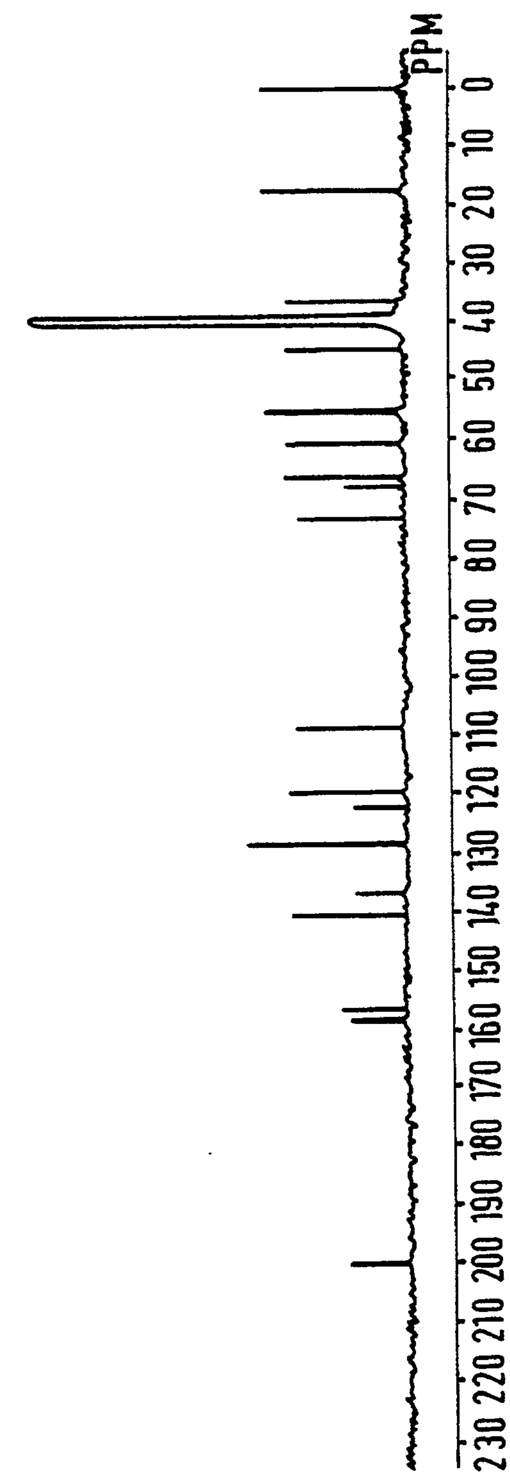
PPM
0
10
20
30
40
50
60
70
80
90
100
110
120
130
140
150
160
170
180
190
200
210
220
230